(19) Europäisches Patentamt
European Patent Office
Office européen des brevets

(11) Publication number : 0 492 930 A2

(12) **EUROPEAN PATENT APPLICATION**

(21) Application number : 91311691.9

(22) Date of filing : 17.12.91

(51) Int. Cl.$^5$ : **A61K 31/135,** A61K 47/20, A61K 9/08

(30) Priority : 20.12.90 US 630480

(43) Date of publication of application : 01.07.92 Bulletin 92/27

(84) Designated Contracting States : AT BE CH DE DK ES FR GB GR IT LI LU NL SE

(71) Applicant : ELI LILLY AND COMPANY
Lilly Corporate Center
Indianapolis Indiana 46285 (US)

(72) Inventor : Valia, Kirti Himatlal
7970 Springwater Drive, West
Indianapolis, Indiana 46256 (US)

(74) Representative : Tapping, Kenneth George et al
Lilly Industries Limited Patent Department Erl Wood Manor
Windlesham Surrey, GU20 6PH (GB)

(54) **Transdermal delivery of dobutamine.**

(57) The present invention provides a method for treating cardiac insufficiency in a warm-blooded animal comprising administering dobutamine transdermally, and formulations suitable therefor.

EP 0 492 930 A2

The present invention relates to a formulation suitable for treating cardiac insufficiency by transdermal application of dobutamine, preferably via a skin patch. The present invention also relates to a method for treating cardiac insufficiency by transdermal application of dobutamine.

Dobutamine, (±)-N-[3-(4-hydroxyphenyl)-1-methylpropyl]-2-(3,4-dihydroxyphenyl)ethylamine, is marketed as the hydrochloride salt under the trademark Dobutrex®. The drug, which is used to treat cardiac insufficiency, is administered by intravenous infusion.

Intravenous infusion of dobutamine has several drawbacks. For example, such method of administration requires inserting a needle, connected to a drug delivery system (typically composed of an infusion pump, a bag containing a solution of dobutamine in a vehicle suitable for intravenous administration and tubing connecting the bag, pump and needle together), into the vein of a patient. To prevent the needle from being dislodged from or tearing the vein the patient's mobility and activities must be severely restricted. Furthermore, unless the drug delivery system contains exceedingly long amounts of tubing, the patient must transport the delivery system with him or her wherever he or she goes. Again, such requirement limits the patient's mobility and/or activities.

Intravenous infusion of dobutamine also is disadvantageous in that administration by such method requires hospitalization, typically in an intensive care ward. Accordingly, administration of dobutamine by intravenous infusion is relatively expensive.

One object of the present invention is to provide a method, and formulation suitable therefor, for using dobutamine to treat cardiac insufficiency which does not require the use of a needle, thereby increasing the patient's mobility and the types of activities which the patient can undertake. A second object of the present invention is to provide a method, and formulation suitable therefor, for using dobutamine to treat cardiac insufficiency which can be utilized at home or on an outpatient basis.

Yet another object of the present invention is to provide a dobutamine formulation which can easily be used by patients requiring intermittent infusion treatment or awaiting heart transplants. A final object of the present invention is to provide a dobutamine formulation which can be easily used by Emergency Medical Technicians while transporting patients with acute exacerbations of heart failure.

It is believed that a method, and formulations suitable therefor, for treating cardiac insufficiency with dobutamine which is capable of achieving the objects of the present invention can be used to improve the quality of life of a patient suffering from cardiac insufficiency or intractable heart failure. Such quality of life improvement is obtained by providing the patient with increased mobility, the ability to undertake activities which could not normally be undertaken by one undergoing intravenous infusion and the opportunity to obtain dobutamine, in the proper dose, without having to be admitted to a hospital.

Other objects, features and advantages of the present invention will become apparent from the subsequent description and the appended claims.

The present invention provides a method for increasing cardiac contractility in a warm-blooded animal which comprises administering through the skin of said animal a cardiac contractility increasing amount of dobutamine, or a pharmaceutically acceptable salt thereof. Administration of dobutamine, or a pharmaceutically acceptable salt thereof, through an animal's skin is typically accomplished using a patch, containing a formulation suitable for transdermal administration, which is placed on the animal's skin. Accordingly, the present invention also provides a formulation suitable for transdermal administration of dobutamine comprising dobutamine, or a pharmaceutically acceptable salt thereof, an alkyl sulfoxide of the formula

$$R^1S(O)R^2,$$

wherein

$R^1$ is $C_4$-$C_{16}$ alkyl, $C_4$-$C_{16}$ alkenyl, $C_4$-$C_{16}$ substituted alkyl or a hetero group containing from 4 to 16 carbon atoms; and

$R^2$ is $C_1$-$C_8$ alkyl or $C_1$-$C_8$ alkylhydroxy, and a pharmaceutically acceptable carrier therefor.

All temperatures stated herein are in degrees Celsius. All units of measurement employed herein are in weight units, unless otherwise noted.

As used herein, a $C_4$-$C_{16}$ alkyl group includes all straight or branched-chain alkyl groups which have from 4 to 16 carbon atoms. Typical $C_4$-$C_{16}$ alkyl groups include n-butyl, sec-butyl, isobutyl, t-butyl, n-pentyl, n-hexyl, isohexyl, heptyl, octyl, nonyl, decyl, undecyl, dodecyl, 2-ethylhexyl, 2-ethyloctyl, 3-propylnonyl and the like.

A $C_4$-$C_{16}$ alkenyl group includes all straight or branched-chain alkenyl groups which have from 4 to 16 carbon atoms. Typical $C_4$-$C_{16}$ alkenyl groupsinclude isobutenyl, 2-methyl-1-butenyl, 2-ethyl-1-butenyl, 2-methyl-2-butenyl, 3-methyl-2-pentenyl, 2,3-dimethyl-1-butenyl, 2,3-dimethyl-2-butenyl, 2-methyl-1-pentenyl, 2-ethyl-1-pentenyl, 2-methyl-2-pentenyl, 3-methyl-2-pentenyl, 3-ethyl-2-pentenyl, 4-methyl-3-heptenyl, pentenyl, hexenyl, heptenyl, octenyl, nonenyl, 3-decenyl, 2-dodecenyl, 3-undecenyl, 3-octenyl and the like.

A $C_4$-$C_{16}$ substituted alkyl group includes all straight or branched-chain alkyl groups which have from 4 to 16 carbon atoms and which are substituted at any position on the alkyl chain with a hydroxy, $C_1$-$C_4$ alkylhydroxy,

$C_1$-$C_4$ alkoxy or keto substituent. Typical $C_4$-$C_{16}$ substituted alkyl groups include 2-ketohexyl, 2-hydroxyheptyl, 2-ketooctyl, 2-ketodecyl, 2-ketoundecyl, 2-ketododecyl, 2-hydroxypentyl, 2-hydroxyheptyl, 2-hydroxyoctyl, 2-hydroxydecyl, 2-hydroxyundecyl, 2-hydroxydodecyl, 3-hydroxyoctyl, 4-hydroxynonyl, 3-hydroxymethylhexyl, 3-hydroxyethyloctyl, 2-methoxyoctyl, 3-methoxyundecyl, 2-methoxydodecyl and the like.

A hetero group containing from 4 to 16 carbon atoms includes all straight and branched-chain alkyl groups which have from 4 to 13 carbon atoms attached to an azole heterocyclic ring. Typical $C_4$-$C_{16}$ hetero groups include 2-hexyl-oxazole, 4-octylthiazole, 3-decyl-pyrazole and the like.

A $C_1$-$C_8$ alkyl group includes all straight or branched-chain alkyl groups which have from one to eight carbon atoms. Typical $C_1$-$C_8$ alkyl groups include methyl, ethyl, n-propyl, isopropyl, n-butyl, sec-butyl, isobutyl, t-butyl, n-pentyl and the like. The term "$C_1$-$C_8$ alkyl" includes within its definition the term "$C_1$-$C_4$ alkyl".

A $C_1$-$C_8$ alkylhydroxy group includes all straight or branched-chain alkyl groups which have from one to eight carbon atoms attached to a hydroxy moiety. Typical $C_1$-$C_8$ alkylhydroxy groups include hydroxymethyl, 2-hydroxyethyl, 1-hydroxypropyl, 3-hydroxypropyl, 4-hydroxybutyl and the like. The term "$C_1$-$C_8$ alkyl-hydroxy" includes within its definition the term "$C_1$-$C_4$ alkylhydroxy".

Finally, a $C_1$-$C_4$ alkoxy group represents methoxy, ethoxy, n-propoxy, isopropoxy, n-butoxy, isobutoxy and tert-butoxy.

The present invention provides a method for increasing cardiac contractility in a warm-blooded animal which comprises administering through the skin of said animal an amount of dobutamine, or a pharmaceutically acceptable salt thereof, sufficient to increase cardiac contractility. To accomplish such method, a formulation capable of delivering sufficiently large quantities of dobutamine transdermally over a sufficiently long period of time had to be devised. The present invention provides such a transdermal formulation.

The first constituent of the present transdermal formulation is, of course, dobutamine or a pharmaceutically acceptable salt thereof. Dobutamine is a racemate of the following structure

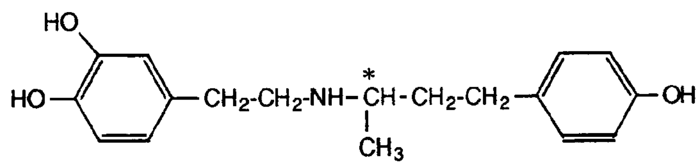

The α-carbon atom (marked with an asterisk) is asymmetric, yielding two stereoisomers, the (+) and (-), which, in a 1:1 mixture, form the racemate. The formulation of the present invention can utilize both the racemate and the (+) and (-) stereoisomers and, accordingly, the term "dobutamine", as used herein, includes both the racemate and its individual stereoisomers. However, the racemate and (-)-stereo-isomer are preferred forms of dobutamine for use in the formulation and method of the present invention.

The formulation of the invention can also employ pharmaceutically acceptable salts of dobutamine. Since dobutamine is an amine, it is basic in nature and, accordingly, will react with any number of pharmaceutically acceptable inorganic and organic acids to form pharmaceutically acceptable acid addition salts. Acids commonly employed to form such salts include inorganic acids such as hydrochloric, hydrobromic, hydroiodic, sulfuric, phosphoric and the like as well as organic acids such as para-toluenesulfonic, methanesulfonic, oxalic, para-bromophenylsulfonic, carbonic, succinic, citric, benzoic, lactic, lactobionic, tartaric and the like. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, hydrochloride, hydrobromide, hydroiodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylenesulfonate, phenylacetate, phenyl-propionate, phenylbutyrate, citrate, lactate, β-hydroxy-butyrate, glycolate, tartrate, lactobionate, methanesulfonate, propanesulfonate, naphthalene-1-sulfonate, p-toluenesulfonate, naphthalene-2-sulfonate, mandalate, gluconate, glucoheptonate, glycerate, malate, mevalonate, dihydroxybutyrate, dihydroxyisobutyrate, dihydroxyvalerate, dihydroxyisovalerate, erythronate, threonate, bis(hydroxymethyl)malonate, 2,3-dihydroxyglutarate, dihydroxyadipate, bis(hydroxymethyl)acetate and the like. The most preferred pharmaceutically acceptable salts of dobutamine which may be employed in the present formulation are the hydrochloride, dihydrogenphosphate, lactate, lactobionate and tartrate salts.

The present transdermal formulation will contain from about 0.1% by weight to about 60.0% by weight of dobutamine. Preferably, the formulation will contain from about 0.5% by weight to about 20.0% by weight of dobutamine. Both of the above ranges are based on the weight percent of active ingredient; namely,

dobutamine free base, present in the formulation since the weight percent of any pharmaceutically acceptable salt of dobutamine present in the formulation will vary greatly depending on what acid is used to prepare the dobutamine salt.

The second constituent of the present transdermal formulation is a pharmaceutically acceptable carrier. The carrier must be capable of dissolving all components of the present formulation and, in particular, must be capable of dissolving dobutamine, or its pharmaceutically acceptable salts, and the alkyl sulfoxide penetration enhancer. In certain instances, the pharmaceutically acceptable carrier may be deleted from the formulation when a low molecular weight liquid alkyl sulfoxide which is capable of dissolving all components of the formulation is employed. In this situation, the alkyl sulfoxide acts as both carrier and penetration enhancer.

Pharmaceutically acceptable carriers suitable for use in the instant formulation include all carriers which are well-known in the medical arts. Suitable carriers include, for example, water, liquid alcohols, liquid glycols and liquid polyalkylene glycols. Accordingly, exemplary carriers include water and alcohols, including both monohydric and polyhydric alcohols, such as ethanol, isopropanol, glycerol, sorbitol, 2-methoxyethanol, diethylene glycol, ethylene glycol, hexylene glycol, mannitol, propylene glycol and the like.

Preferably, the carrier employed in the instant formulation will be water, a $C_2$-$C_6$ alcohol or a mixture thereof. Suitable alcohols include ethanol, isopropanol, hexanol and the like. Especially preferred carriers are water and water/ethanol mixtures of from about 1:20 to about 5:1 by weight water to ethanol. The most preferred carriers are water and about a 1:1 ethanol/water mixture.

The carrier will be present in the instant formulation in an amount of from about 30.0% by weight to about 99.8% by weight. Preferably, the carrier will be present in an amount of from about 75.0% by weight to about 99.0% by weight. Again, as for the concentration ranges for dobutamine, both of the above ranges are based on the weight percent of dobutamine free base present in the formulation.

In order to effectively increase cardiac contractility in a warm-blooded animal, about 0.5 to about 10.0 mcg/kg of body weight per minute (more preferably about 2.5 to about 10.0 mcg/kg of body weight per minute) of dobutamine must be delivered to the patient's circulatory system. When formulations consisting solely of dobutamine, or a pharmaceutically acceptable salt thereof, in combination with a carrier, as described above, are applied to the skin of a warm-blooded animal insufficient quantities of dobutamine are transferred through the skin to the animal's circulatory system. Accordingly, in order to have a formulation suitable for transdermal administration of dobutamine such formulation must contain an additional constituent. This additional constituent is an alkyl sulfoxide penetration enhancer.

Accordingly, the final required constituent of the present transdermal formulation is an alkyl sulfoxide of the formula

$$R^1S(O)R^2$$

wherein

$R^1$ is $C_4$-$C_{16}$ alkyl, $C_4$-$C_{16}$ alkenyl, $C_4$-$C_{16}$ substituted alkyl or a hetero group containing from 4 to 16 carbon atoms, and

$R^2$ is $C_1$-$C_8$ alkyl or $C_1$-$C_8$ alkylhydroxy. Preferred alkyl sulfoxides are those wherein $R^1$ is $C_8$-$C_{12}$ alkyl or $C_8$-$C_{12}$ hydroxy substituted alkyl and $R^2$ is as defined above.

Particularly preferred alkyl sulfoxides for use in the present formulation are those sulfoxides in which $R^1$ is $C_8$-$C_{12}$ alkyl or $C_8$-$C_{12}$ hydroxy substituted alkyl and $R^2$ is $C_1$-$C_8$ alkyl. Especially preferred sulfoxides for purposes of the present invention are those sulfoxides wherein $R^1$ is $C_8$-$C_{12}$ alkyl or $C_8$-$C_{12}$ hydroxy substituted alkyl and $R^2$ is $C_1$-$C_3$ alkyl. Examples of such especially preferred sulfoxides include octyl methyl sulfoxide, nonyl methyl sulfoxide, decyl methyl sulfoxide, undecyl methyl sulfoxide, dodecyl methyl sulfoxide, 2-hydroxydecyl methyl sulfoxide, 2-hydroxy-undecyl methyl sulfoxide, 2-hydroxydodecyl methyl sulfoxide and the like. Decyl methyl sulfoxide is the most preferred penetration enhancer for use in the transdermal formulation of the present invention.

The alkyl sulfoxide will be present in the instant formulation in an amount of from about 0.1% by weight to about 10.0% by weight. Preferably, the sulfoxide will be present in an amount of from about 0.5% by weight to about 5.0% by weight, and even more preferably from about 0.5% to about 1.5% by weight. The sulfoxide compounds disclosed above can be used singly or in combination in the instant formulation. As for both the dobutamine and carrier ranges discussed above, all of the above sulfoxide ranges are based on the weight percent of dobutamine free base present in the formulation.

While not required, the formulation of the invention can also include various ingredients commonly employed in transdermal formulations. For example, thickening agents such as carboxymethylcellulose, hydroxypropylmethylcellulose and the like and anti-oxidants such as the alkali metal sulfites or bisulfites, thioglycerol, thioerithritol and the like can be present in the formulation in order to ensure stability and provide a more pleasing aesthetic appearance.

The formulation of the present invention is prepared by blending the above-mentioned ingredients together

until a homogeneous solution is obtained. This solution may then be used to increase cardiac contractility in a warm-blooded animal by applying it directly to the skin of the animal to be treated. Alternatively, and preferably, the solution can also be placed inside a transdermal patch and then the patch may be placed on the skin of the animal to be treated. Transdermal patches suitable for use with the present formulation are well known to those skilled in the art. Examples of such patches include polyvinyl alcohol hydrogel sheets from Dow Corning and cellulose triacetate polymer patches from Moleculon.

To effectively increase cardiac contractility in a warm-blooded animal, about 0.5 to about 10.0 mcg/kg of body weight per minute (more preferably about 2.5 to about 10.0 mcg/kg of body weight per minute) of dobutamine must be delivered to the patient's circulatory system. The present invention provides a method for increasing cardiac contractility comprising delivering dobutamine transdermally to a patient's circulatory system. Control of the dosage of dobutamine delivered to the patient's circulatory system under the present transdermal method, may be accomplished by limiting the amount of formulation applied directly to the skin of the patient or by limiting the amount of formulation inserted into the transdermal patch. Alternatively, the dosage of dobutamine administered may be controlled by limiting the surface area of the transdermal patch or by limiting the surface area to which the formulation is directly applied.

All of the materials employed in the formulation of the present invention are either commercially available or can be prepared by processes well-known in the art. For example, dobutamine, and numerous pharmaceutically acceptable salts thereof, are described in United States Patent Nos. 3,987,200 and 4,704,407 and European Patent Application 322149. The alkyl sulfoxides used herein include those compounds described in United States Patent Nos. 3,527,864, 3,551,554 and 3,896,238. The teachings of the above-mentioned patents and patent application are herein incorporated by reference.

The following Examples further illustrate specific aspects of the present invention, including specific formulations and their ability to deliver sufficiently large amounts of dobutamine transdermally. It is to be understood, however, that the Examples are included for illustrative purposes only and are not intended to limit the scope of the invention in any respect and should not be so construed.

In Vitro Studies

The ability of the formulation of the invention to provide transdermal penetration of dobutamine was shown by measuring the diffusion of dobutamine through full thickness human cadaver skin using the in vitro diffusion cell permeation apparatus and method described by Valia et al. in Drug Development and Industrial Pharmacy, 10(7), 951-981 (1984) which is incorporated herein by reference. Briefly, the apparatus used was a skin permeation cell consisting of two cylindrical half-cells in mirror image. Each of the half-cells was composed of a solution compartment which was enclosed inside of a water jacket compartment. Each of the half-cells was equipped with a closable sampling port and a depression in the solution compartment which serves as the platform for a star-head rotating magnet. The magnets stirred the solution in the half-cells at a constant rate of 600 revolutions per minute by an external synchronous driving unit. Both donor and receptor compartments were thermostatically controlled at a constant temperature by circulating 37°C water throught the water jacket compartment by means of an external circulator. The skin sample (human cadaver skin) used in the investigations was then mounted between the two half-cells. In the donor compartment was placed the various test solutions described below and in the receptor compartment was placed carrier fluid alone. During the diffusion period (usually from about 0 to about 50 hours) samples were withdrawn periodically from the receptor compartment and the amount of dobutamine in the sample (corresponding to the amount of dobutamine which had diffused across the skin) was determined using the high performance liquid chromatography (HPLC) assay set forth below.

The HPLC assay used to determine the amount of dobutamine in the sample employed a μBondapak C18 column. The column was eluted with a mobile phase consisting of a 3:2 0.02M potassium phosphate monobasic (pH 4.0)/methanol solution. The column detector had a wavelength of 281 nm, the column flow rate was 1.0 ml/min, the injection volume was 10 μl and the column temperature was ambient.

In Table 1, below, the permeation rates of solutions containing 14 mg of dobutamine hydrochloride (12.5 mg equivalents of dobutamine free base), 0.25 mg of sodium bisulfite, 1 ml of water and various amounts of n-decyl methyl sulfoxide are set forth. Column 1 of the table shows the concentration, by weight, of sulfoxide present in the solution (calculated on a dobutamine free base weight basis). Column 2 shows the permeation rate of dobutamine obtained in mcg/cm$^2$/hr. Finally, Column 3 discloses the approximate amplification over control (formulation containing 0% decyl methyl sulfoxide) obtained when various amounts of n-decyl methyl sulfoxide were added to the test solution.

Table 1

| Conc. n-decyl methyl sulfoxide (%) | Permeation Rate $mcg/cm^2/hr$ | Approximate Amplification Over Control |
|---|---|---|
| 0 | 0.18 ± 0.06 | -- |
| 0.98 | 315.62 ± 8.91 | 1750 |
| 0.98 | 286.66 ± 37.12 | 1590 |
| 4.70 | 194.53 ± 28.65 | 1080 |

In Table 2, below, the permeation rates of solutions containing 219 mg of dobutamine lactobionate (100.0 mg equivalents of dobutamine free base), 2.0 mg of sodium bisulfite, 1 ml of water and various amounts of n-decyl methyl sulfoxide are set forth. Columns 1, 2 and 3 of Table 2 contain the same types of information as described above for Columns 1, 2 and 3 of Table 1.

Table 2

| Conc. n-decyl methyl sulfoxide (%) | Permeation Rate $mcg/cm^2/hr$ | Approximate Amplification Over Control |
|---|---|---|
| 0 | 0.88 ± 0.44 | -- |
| 0.90 | 913.87 ± 257.81 | 1040 |

In Table 3, below, the permeation rates of solutions containing 10 mg of n-decyl methyl sulfoxide, 1 ml of water and various amounts of dobutamine lactobionate [both the racemate and the (-) stereoisomer] and sodium bisulfite are set forth. Column 1 of Table 3 shows the amount of dobutamine lactobionate in the solution. Columns 2 and 3, respectively, of that table show the amount of dobutamine free base equivalents present in the solution and whether the racemate or the (-)-stereoisomer was tested. Column 4 of Table 3 shows the amount of sodium bisulfite present in the test solution, while Column 5 discloses the permeation rate of dobutamine obtained in $mcg/cm^2/hr$.

Table 3

| Dobutamine Lactobionate (mg) | Free Base Eq. (mg) | Stereo-chemistry | Sodium Bisulfite (mg) | Permeation Rate $mcg/cm^2/hr$ |
|---|---|---|---|---|
| 22 | 9.5 | racemate | 0.2 | 113 ± 4 |
| 55 | 21.5 | racemate | 0.5 | 261 ± 13 |
| 110 | 45.0 | racemate | 1.0 | 527 ± 35 |
| 220 | 83.5 | racemate | 2.0 | 804 ± 80 |
| 440 | 153.8 | racemate | 4.0 | 930 ± 161 |
| 22 | 9.5 | (-)isomer | 0.2 | 149 ± 28 |
| 55 | 21.5 | (-)isomer | 0.5 | 317 ± 62 |
| 110 | 45.0 | (-)isomer | 1.0 | 502 ± 82 |
| 220 | 83.5 | (-)isomer | 2.0 | 542 ± 178 |
| 440 | 153.8 | (-)isomer | 4.0 | 523 ± 118 |

In Table 4, below, the permeation rates of solutions containing 10 mg of n-decyl methyl sulfoxide, 1 ml of water and various amounts of dobutamine dihydrogenphosphate and sodium bisulfite are set forth. Column 1 of Table 4 shows the amount of dobutamine dihydrogen phosphate present in the test solution. Columns 2 and 3, respectively, of that table show the amount of dobutamine free base equivalents and the amount of sodium bisulfite present in the solution. Column 5 of Table 4 discloses the permeation rate of dobutamine obtained in $mcg/cm^2/hr$.

Table 4

| Dobutamine Dihydrogen Phosphate (mg) | Free Base Eq (mg) | Sodium Bisulfite (mg) | Permeation Rate ($mcg/cm^2/hr$) |
|---|---|---|---|
| 132.5 | 100 | 2.0 | 406.9 ± 57.42 |
| 265.0 | 200 | 4.0 | 502.84 ± 4.93 |

As can be seen from Tables 1 through 4, above, when formulations containing just a salt of dobutamine and water are employed, insignificant quantities of dobutamine can be transferred across the skin. However, when n-decyl methyl sulfoxide is added to the formulation dobutamine skin penetration increases dramatically.

## In Vivo Studies

To corroborate the in vitro results obtained using formulations of the present invention, the ability of the present formulation to provide transdermal delivery of dobutamine lactobionate through the skin of a living dog was also studied. The methodology and results obtained from such in vivo study is reported below.

Two male beagle dogs were anesthetized with 100 mg/kg of sodium phenobarbitol and prepared for monitoring of electrocardiogram, contractile tension, heart rate and mean arterial pressure. Dobutamine lactobionate was administered by an i.v. bolus (4, 8 and 16 mcg/kg). After each animal's response returned to baseline, 1.0 ml of a formulation containing 218.9 mg of dobutamine lactobionate (100 mg equivalents of dobutamine free base), 2.0 mg of sodium bisulfite, 10.0 mg of n-decyl methyl sulfoxide in 1 ml of water was applied to a 25 $cm^2$ shaved area in the groin-abdominal region of each dog. Cardiac contractility changes in each dog were then monitored for the next 3 to 5 hours.

Table 5, below, reports the results obtained in the above described in vivo study. In Table 5, Column 1 shows the method used to apply the formulation to each animal [Method I - formulation applied directly to the animal's skin and then covered with Saran Wrap plastic film; Method II - formulation placed inside a 5 cm x 0.0508 cm cellulose triacetate polymer patch (Moleculon, POROPLASTIC PATCH MA-92) which patch is then applied to the animal's skin]. Column 2 discloses the time elapsed from the time the formulation was applied and Column 3 discloses the percent change in contractility (from baseline) observed at the time value shown in Column 2.

Table 5

| Application Method | Time (min) | % Change In Contractility |
|---|---|---|
| Method I | 10 | 16 |
| | 20 | 30 |
| | 30 | 35 |
| | 60 | 48 |
| | 90 | 21 |
| | 120 | 16 |
| | 180 | 0 |
| Method II | 10 | 10 |
| | 20 | 26 |
| | 30 | 24 |
| | 60 | 36 |
| | 90 | 41 |
| | 120 | 33 |
| | 150 | 25 |
| | 180 | 27 |
| | 210 | 22 |
| | 240 | 9 |
| | 270 | 9 |
| | 300 | 11 |

As can be seen from Table 5 above, the formulation of the present invention can be used to deliver pharmacologically effective amounts of dobutamine through the skin of a live animal. Accordingly, it is believed the formulation of the present invention can be used to provide a method for increasing cardiac contractility in a warm-blooded animal which comprises administering through the skin of said animal a cardiac contractility increasing amount of dobutamine, or a pharmaceutically acceptable salt thereof. As noted before, as used herein, the term "a cardiac contractility increasing amount of dobutamine, or a pharmaceutically acceptable salt thereof" refers to a dosage range of from about 0.5 to about 10.0 mcg/kg of body weight per minute (more preferably from about 2.5 to about 10.0 mcg/kg of body weight per minute) of dobutamine free base delivered to the circulatory system of the animal being treated.

The method of the present invention, which requires transdermal administration of dobutamine (and the formulation required thereby), is believed capable of providing substantial advantages in terms of improving the quality of life of a patient suffering from cardiac insufficiency or intractable heart failure. The present method allows dobutamine to be administered at home or on an out-patient basis, thereby minimizing or eliminating the patient's hospital stay. Furthermore, since the present method applies dobutamine to the patient's skin, the cumbersome equipment associated with intravenous administration of dobutamine is not required. Once such cumbersome equipment is eliminated the patient's mobility and types of activities which can be undertaken significantly increase, thereby providing significant improvement in the patient's "quality of life".

## Claims

1. A formulation suitable for transdermal administration of dobutamine comprising dobutamine, or a pharmaceutically acceptable salt thereof, an alkyl sulfoxide of the formula

$$R^1S(O)R^2,$$

wherein

$R^1$ is $C_4$-$C_{16}$ alkyl, $C_4$-$C_{16}$ alkenyl, $C_4$-$C_{16}$ substituted alkyl or a hetero group containing from 4 to 16 carbon atoms; and

$R^2$ is $C_1$-$C_8$ alkyl or $C_1$-$C_8$ alkylhydroxy, and a pharmaceutically acceptable carrier therefor.

2. A formulation of Claim 1 wherein racemic dobutamine, or a pharmaceutically acceptable salt thereof, is employed.

3. A formulation of Claim 1 or 2 wherein the pharmaceutically acceptable salt of dobutamine is dobutamine hydrochloride.

4. A formulation of Claim 1 or 2 wherein the pharmaceutically acceptable salt of dobutamine is dobutamine lactobionate.

5. A formulation of any one of Claims 1 to 4 wherein the pharmaceutically acceptable carrier is water, a $C_2$-$C_6$ alcohol or a mixture thereof.

6. A formulation of any one of Claims 1 to 5 wherein the carrier is water.

7. A formulation of any one of Claims 1 to 5 wherein the carrier is a 1:1 (w/w) ethanol/water mixture.

8. A formulation of any one of Claims 1 to 7 wherein the alkyl sulfoxide employed has an $R^1$ substituent which is $C_8$-$C_{12}$ alkyl or $C_8$-$C_{12}$ hydroxy substituted alkyl and an $R^2$ substituent which is $C_1$-$C_8$ alkyl or $C_1$-$C_8$ alkylhydroxy.

9. A formulation of any one of Claims 1 to 8 wherein the alkyl sulfoxide employed has an $R^2$ substituent which is $C_1$-$C_3$ alkyl.

10. A formulation as claimed in any one of Claims 1 to 9 for use in increasing cardiac contractility in a warm-blooded animal.

**Claims for the following Contracting States: GR, ES**

1. A process for preparing a formulation suitable for transdermal administration of dobutamine comprising admixing dobutamine, or a pharmaceutically acceptable salt thereof, an alkyl sulfoxide of the formula

$$R^1S\ (O)\ R^2,$$

wherein $R^1$ is $C_4$-$C_{16}$ alkyl, $C_4$-$C_{16}$ alkenyl, $C_4$-$C_{16}$ substituted alkyl or a hetero group containing from 4 to 16 carbon atoms and

$R^2$ is $C_1$-$C_8$ alkyl or $C_1$-$C_8$ alkylhydroxy, and a pharmaceutically acceptable carrier.

2. A process of Claim 1 wherein racemic dobutamine, or a pharmaceutically acceptable salt thereof, is employed.

3. A process of Claim 1 or 2 wherein the pharmaceutically acceptable salt of dobutamine is dobutamine hydrochloride.

4. A process of Claim 1 or 2 wherein the pharmaceutically acceptable salt of dobutamine is dobutamine lactobionate.

5. A process of any one of Claims 1 to 4 wherein the pharmaceutically acceptable carrier is water, a $C_2$-$C_6$ alcohol or a mixture thereof.

6. ! process of any one of Claims 1 to 5 wherein the carrier is water.

7. A process of any one of Claims 1 to 5 wherein the carrier is a 1:1 (w/w) ethanol/water mixture.

8. A process of any one of Claims 1 to 7 wherein the alkyl sulfoxide employed has an $R^1$ substituent which is $C_8$-$C_{12}$ hydroxy substituted alkyl and an $R^2$ substituent which is $C_1$-$C_6$ alkyl or $C_1$-$C_8$ alkylhydroxy.

9. A process of anh one of Claims 1 to 8 wherein the alkyl sulfoxide employed has an $R^2$ substituent which is $C_1$-$C_3$ alkyl.